Europäisches Patentamt

European Patent Office

Office européen des brevets

(19)

(11) Publication number: **0 313 703**
**B1**

# (12) EUROPEAN PATENT SPECIFICATION

(45) Date of publication of the patent specification:
26.09.90

(51) Int. Cl.⁵: **A61K 31/59**

(21) Application number: 87309643.2

(22) Date of filing: 30.10.87

(54) Use of 24,25-dihydroxycholecalciferole in the treatment of urinary calculus.

(43) Date of publication of application:
03.05.89 Bulletin 89/18

(45) Publication of the grant of the patent:
26.09.90 Bulletin 90/39

(84) Designated Contracting States:
CH DE FR GB IT LI SE

(73) Proprietor: KUREHA KAGAKU KOGYO KABUSHIKI
KAISHA, 9-11 Horidome-cho 1-chome Nihonbashi
Chuo-ku, Tokyo 103(JP)

(72) Inventor: Yamato, Hideyuki, 5-24-21 Takenotsuka,
Adachi-ku, Tokyo(JP)
Inventor: Maeda, Yuji,
2-609 Minaminagareyama-Ichibangai, 2013 Nagareyama,
Nagareyama-shi, Chiba-ken(JP)
Inventor: Fujii, Takayoshi, 5-11-21 Towa, Adachi-ku,
Tokyo(JP)
Inventor: Kobayashi, Yasuhiko, 1-2-10 Shinbori,
Niiza-shi, Saitama-ken(JP)
Inventor: Saito, Kenichi,
204 Kureha-Kagaku-Okubo-Shataku,
3-26-1 Hyakunin-cho Shinjuku-ku, Tokyo(JP)
Inventor: Hirose, Fumio,
22 Kureha-Kagaku-Ichigaya-So, 8 Ichigayadai-machi,
Shinjuku-ku, Tokyo(JP)
Inventor: Kato, Tadaaki, K-407, 3-27 Nakadai,
Itabashi-ku, Tokyo(JP)
Inventor: Yoshikumi, Chikao, 2-19-46 Higashi,
Kunitachi-shi, Tokyo(JP)

(74) Representative: Woods, Geoffrey Corlett et al, J.A.
KEMP & CO. 14 South Square Gray's Inn, London
WC1R 5EU(GB)

(56) References cited:
EP-A- 0 086 476

J. LAB. CLIN. MED., vol. 91, no. 5, 1978, page 840-849,
The C.V. Mosby Co.; A.E. CLADAS et al.: "The
simultaneous measurement of vitamin D metabolites in
plasma: studies in healthy adults and in patients with
calcium nephrolithiasis"
ARCH. INTERN. MED., vol. 145, April 1985,
pages 681-684; J.C. NETELENBOS et al.: "Vitamin D
status in urinary calcium tone formation"
ANN. NUTR. METAB., vol. 29, 1985, pages 289-296, S.
Karger AG, Basel, CH; M. GASCON-BARRE et al.:
"Interrelationships between circulating vitamin D
metabolites in normocalciuric and hypercalciuric renal
stone formers"
AVIATION, SPACE, AND ENVIRONMENTAL MEDICINE,
vol. 54, no. 5, May 1983, pages 447-451, Aerospace
Medical Association, Washington, DC; A.S. USHAKOV
et al.: "Bone tissue of hypokinetic rats: effects

(56) References cited: (continuation)
of 24,25-dihydroxycholecalciferol and varying
phosphorous content in the diet"o. 131 178als of rat
liver", & BIOCHIM. BIOPHYS. ACTA 1985, 842(1), 12-21
000
THERAPIEWOCHE, vol. 29, 1979, pages 2137-2141,
Verlag G. BRAUN, Karlsruhe, DE; H. SCHMIDT-GAYK et
al.: "Vitamin-D-Stoffwechsel bei Urolithiasis: neuere
Befunde und Untersuchungsmethoden"

## Description

The present invention relates to a the use of 24,25-dihydroxycholecalciferol in the treatment of urinarycalculus.

The formation of urinary calculus is a disease of high frequency among the diseases of the urinary system. The words "urinary calculus" herein mentioned include renal calculus, prostatic calculus, renal pelvic calculus, ureteric calculus, vesical calculus and urethral calculus.

Although the above disease is treated, at present in many cases, by the operative extirpation of the calculus, there are problems. The patient experiences pain during the operation. Hospitalisation is required.

Recently, the establishment of an internal medical method for treating urinary calculus has been regarded as a very important problem. The development of a prophylactic for urinary calculus has been desired.

As a result of the present inventors' studies on the pharmacological effects of various substances, which exist in a healthy human body and have a verified safety, to the urinary calculus, it has been found that 24,25-dihydroxycholecalciferol (hereinafter referred to as the present substance or 24,25-$(OH)_2$-$D_3$) remarkably inhibits the formation of urinary calculus in an experiment using model animals, and on the basis of the finding, the present invention have been accomplished.

In a first aspect of the present invention, there is provided a use of 24,25-dihydroxycholecalciferol for the manufacture of a pharmaceutical composition for inhibiting the formation of urinary calculus.

In a second aspect of the present invention, there is provided a use of 24,25-dihydroxycholecalciferol for the manufacture of a pharmaceutical composition for prophylaxis or urinary calculosis.

Every one of the present substances has been known and for instance, the present substances have been disclosed in Pharmacia, 10, 319 - 322(1974).

The present substance includes 24R,25-$(OH)_2$-$D_3$, 24S, 25-$(OH)_2$-$D_3$ or a mixture thereof, and in particular, it is preferable to use 24R,25-$(OH)_2$-$D_3$ as the present substance.

In the present invention, although 24,25-$(OH)_2$-$D_3$ may be preferably administered parenterally, it may also be administered orally.

The pharmaceutical preparation containing the present substance as a pharmaceutical active ingredient can take various dosage forms such as tablet, powder, granule, suppository, capsule, alcoholic solution, oily solution and aqueous suspension. As the oily solvent, trigylceride of a fatty acid of 8 to 10 carbon atoms, corn oil, cotton seed oil, peanut oil, fish liver oil and oily ester are used. Cacao oil and glycerol are also usable. As the other component, lactose, starch, talc, magnesium stearate, sorbic acid, salts of sorbic acid, saccharides or derivatives thereof, alcohol, aqueous physiological saline solution, surfactant, antioxidant, etc. may be used together with the present substance.

The present substance may be contained in the pharmaceutical preparation in an amount of from 0.00002 to 4 % by weight, preferably from 0.0002 to 1 % by weight.

Further, the present substance is administered to an adult in a daily dose of 0.1 to 100,000μg, preferably 0.5 to 10,000 μg.

The results of examination on the acute toxicity of the present substance are shown as follows.

Acute toxicity:

After dissolving the present substance in ethanol, the thus formed solution was diluted by a triglyceride of a fatty acid of 8 to 10 carbon atoms so that the concentration of ethanol became 2 %. The thus formed solution was orally (p.o.) administered to each of 10 male ICR mice (age of 6 weeks, body weight of 25±3 g) at dose of 150 mg/kg. On observing the presence of toxic symptoms on the animals for 2 weeks, all the animals (10 mice) survived without any abnormality. After slaughtering the test animals, the biochemical examination of the blood of the animals, the autopsy and the histopathological examination were carried out, however, no difference was found between the results for the test animals and the results for the control animals which were administered with only the triglyceride of a fatty acid of 8 to 10 carbon atoms containing 2 % of ethanol. Accordingly, the acute oral $LD_{50}$ of the present substance is not less than 100 mg/kg and the present substance can be said to be extremely safe.

The present inventors examined the effect of 24,25-$(OH)_2$-$D_3$ on inhibiting the formation of urinary calculus using model animals and have found that the present substance is effective as a prophylactic for urinary calculosis.

The present invention will be more precisely explained while referring to the following non-limitative Examples. The absolute configuration of 24- position of 24, 25-$(OH)_2$-$D_3$ was identified while referring to Tetrahedron Letters No. 26, 2203 - 2206, 1975.

### EXAMPLE 1:

As the test animals for renal calculus, the following rats were used.

Twenty male Wistar rats (age of 8 weeks and body weight of about 200 g) were allowed to take a water containing 0.5 % of ethylene glycol freely, and a mixture of 0.5 μg of 1α-hydroxycholecalciferol and 0.1 ml of triglyceride of a fatty acid of 8 to 10 carbon atoms was forcibly and orally administered by a stomach tube every other day for four weeks, thereafter being used as the test animals.

24R,25-$(OH)_2$-$D_3$ in the triglyceride of a fatty acid of 8 to 10 carbon atoms containing 2 % of ethanol was forcibly and orally administered to each of the test animals at a dose of 10μg/kg/1 ml/day by a stomach tube, and to each animals of the control group, the triglyceride of a fatty acid of 8 to 10 carbon atoms was administered in the same manner as above.

The animals were slaughtered after four weeks from the first administration, and the kidney of the animal was extirpated therefrom after the exsanguination and immediately photographed by soft X-ray. Furhter the cross section of the extirpated kidney containing papilla was observed by naked eyes.

Results:

(1) Results of the observation by naked eyes:

In the control group, on the 8 cases among 10 rats, the calculi were observed in the pelvis and the papilla of the kidney, and furthermore a radial deposition of crystals was remarkably found in the renal parenchyma through the interface between the cortex and the medulla to the medulla and the papilla. However, in the group administered with 24R,25-$(OH)_2$-$D_3$ at a dose of 10 µg/kg/1 ml/day, although the radial deposition of crystals was recognized in only one rat of 10 rats, any clear calculus was never recognized.

(2) Soft X-ray photograph:

A clear inhibition of the formation of calculus was found in the rats which were administered with 24R,25-$(OH)_2$-$D_3$ as compared in the control rats.

EXAMPLE 2:

After dissolving 24R,25-$(OH)_2$-$D_3$, at a prescribed concentration, into the triglyceride of a fatty acid of 8 to 10 carbon atoms containing 1 % of ethanol, the solution was forcibly and orally administered to each of the three groups of the male or female ICR mice continuously for 30 days at a daily dose of 10, 100 or 1000 µg/kg.

According to the growth curve of the thus treated animals based on the body weight, any significant difference in the body weight change was not recognized between the groups.

After fixing the following organs by an aqueous 10 % solution of formalin, the thus fixed organs were stained with hematoxylin·eosin and examined histopathologically, however, any abnormal findings were not particularly recognized.

The examined organs are as follows:
brain, heart, lung, liver, kidney, adrenal, spleen, pancreas, thyroid, hypophysis, thymus, mesentric lymph node, testis, ovary, uterus, stomach, small intestines (jejunum, ileum and duodenum), large intestines (colon and cecum), eyeball, submandibular gland, urinary bladder, backskin, muscle, sternum, marrow of sternum, femur and marrow of femur.

EXAMPLE 3:

The triglyceride of a fatty acid of 8 to 10 carbon atoms was irradiated with ultraviolet rays from a 400W high pressure mercury lamp for 72 hours under argon bubbling to decompose the reactive peroxides contained therin as impurities. A solution was prepared by dissolving 5 mg of 24R,25-$(OH)_2$-$D_3$ into 1 kg of the thus treated triglyceride.

Soft capsule containing 0.5 µg of 24R,25-$(OH)_2$-$D_3$ in each capsule was prepared by heating and dissolving the following components for the capsular skin with a soft capsule-producing machine according to an ordinary method.

Recipe of the capsular skin based on the capsule prepared
Gelatine 10 parts by weight
Glycerol 2 parts by weight
Antiseptic (ethylparaben) 0.05 part by weight
Titan white 0.2 part by weight
Water 0.2 part by weight

In the same manner, each soft capsule respectively containing 1, 2, 5 and 10 µg of 24R,25-$(OH)_2$-$D_3$ in one capsule were prepared.

**Claims**

1. Use of 24,25-dihydroxycholecalciferol in the manufacture of a pharmaceutical composition for use in the treatment of urinary calculus.

2. Use according to claim 1, wherein the pharmaceutical composition is for use in inhibiting the formation of urinary calculus.

3. Use according to claim 1, wherein the pharmaceutical composition is for use in the prophylactic treatment of urinary calculus.

4. Use according to any one of the preceding claims, wherein the said 24,25-dihydroxycholecalciferol is 24R,25-dihydroxycholecalciferol.

5. Use according to any one of claims 1 to 3, wherein the said 24,25-dihydroxycholecalciferol is 24S,25-dihydroxycholecalciferol.

6. Use according to any one of claims 1 to 3, wherein a mixture of 24R,25-dihydroxycholecalciferol and 24S,25-dihydroxycholecalciferol is employed.

**Patentansprüche**

1. Verwendung von 24,25-Dihydroxycholecalciferol zur Herstellung einer pharmazeutischen Zubereitung zur Verwendung bei der Behandlung von Blasensteinen.

2. Verwendung nach Anspruch 1, worin die pharmazeutische Zubereitung für die Inhibierung der Bildung von Blasensteinen verwendet wird.

3. Verwendung nach Anspruch 1, worin die pharmazeutische Zubereitung zur prophylaktischen Behandlung von Blasensteinen verwendet wird.

4. Verwendung nach einem der vorhergehenden Ansprüche, worin das 24,25-Dihydroxycholecalciferol 24R,25-Dihydroxycholecalciferol ist.

5. Verwendung nach einem der Ansprüche 1 bis 3, worin das 24,25-Dihydroxycholecalciferol 24S,25-Dihydroxycholecalciferol ist.

6. Verwendung nach einem der Ansprüche 1 bis 3, worin eine Mischung von 24R,25-Dihydroxycholecalciferol und 24S,25-Dihydroxycholecalciferol verwendet wird.

**Revendications**

1. Utilisation du 24,25-dihydroxycholécalciférol dans la fabrication d'une composition pharmaceutique destinée à être utilisée dans le traitement du calcul urinaire.

2. Utilisation selon la revendication 1, dans laquelle la composition pharmaceutique est destinée à être utilisée dans l'inhibition de la formation du calcul urinaire.

3. Utilisation selon la revendication 1, dans laquelle la composition pharmaceutique est destinée à être utilisée dans le traitement préventif du calcul urinaire.

4. Utilisation selon l'une quelconque des revendications précédentes dans laquelle le 24,25-dihydroxycholécalciférol est le 24R,25-dihydroxycholécalciférol.

5. Utilisation selon l'une quelconque des revendications 1 à 3, dans laquelle le 24,25-dihydroxycholécalciférol est le 24S,25-dihydroxycholécalciférol.

6. Utilisation selon l'une quelconque des revendications 1 à 3 dans laquelle on utilise un mélange de 24R,25-dihydroxycholécalciférol et de 24S,25-dihydroxycholécalciférol.